# EUROPEAN PATENT SPECIFICATION

(11) **EP 2 436 304 B1**
(45) Date of publication and mention of the grant of the patent: **28.01.2015**
(21) Application number: 11183047.7
(22) Date of filing: 28.09.2011
(51) Int. Cl.: A61B 1/06, H04N 5/225, A61B 1/00, A61B 1/045, H04N 5/235

(54) **Endoscope device**
Endoskopvorrichtung
Dispositif d'endoscope

(30) Priority: 29.09.2010 JP 2010219282
(43) Date of publication of application: 04.04.2012
(73) Proprietor: FUJIFILM Corporation, Tokyo (JP)
(72) Inventor: Yamaguchi, Hiroshi, Kanagawa (JP); Minetoma, Yasuhiro, Kanagawa (JP)
(74) Representative: Klunker . Schmitt-Nilson . Hirsch

(56) References cited:
- EP-A2- 1 980 199
- EP-A2- 2 020 202
- EP-A2- 2 179 687
- WO-A1-2009/128391
- US-B2- 6 582 362

## Description

### BACKGROUND OF THE INVENTION

The present invention relates to an endoscope device capable of performing a special light observation using specific narrow band light and wide band light such as white illumination light.

In recent years, an endoscope device capable of performing a so-called special light observation has been used, where the special light observation obtains information on a tissue at a desired depth of a living body by emitting specific narrow band light (narrow band light) to a mucous tissue of the living body. This type of endoscope device may simply visualize living body information, which cannot be obtained from an ordinary observation image, by emphasizing a lesion and a microstructure of a surface layer of a new blood vessel generated at, for example, a mucous layer or a lower mucous layer. For example, when an observation subject is a cancer lesion, if narrow band blue light (B) is emitted to the mucous layer, the microstructure or the microscopic blood vessel of the surface layer of the tissue may be observed in more detail, so that the lesion may be more accurately diagnosed.

On the other hand, an invasion depth of light in the thickness direction of the living body tissue is dependent on the wavelength of the light. In the case of the blue light (B) having a short wavelength, the light only reaches the vicinity of the surface layer due to the absorbing and scattering characteristics of the living body tissue, and is absorbed and scattered at the depth range, so that the light may be observed as returned light mainly including information on the surface layer tissue. In the case of green light G having a wavelength longer than that of the B light, the light reaches a position deeper than the range the B light reaches, and is absorbed and scattered at this range, so that the light may be observed as returned light mainly including information on the intermediate layer tissue and the surface layer tissue. In the case of red light (R) having a wavelength longer than that of the G light, the light reaches a deeper position of the tissue, and is absorbed and scattered at this range, so that the light may be observed as returned light mainly including information on the deep layer tissue and the intermediate layer tissue.

That is, image signals obtained by receiving light using an imaging sensor such as a CCD after emitting the B light, the G light, and the R light respectively mainly include information on the surface layer tissue, information on the intermediate layer tissue and the surface layer tissue, and information on the deep layer tissue and the intermediate layer tissue.

For this reason, in the special light observation, in order to easily observe the microstructure or the microscopic blood vessel of the tissue surface layer of the living body tissue, only two types of narrow band light, that is, the narrow band light of blue (B) suitable for observing the surface layer tissue and the narrow band green light G suitable for observing the intermediate layer tissue and the surface layer tissue are used as the narrow band light emitted to the living body tissue without using the narrow band red light R mainly suitable for observing the intermediate layer tissue and the deep layer tissue of the living body tissue. Then, image processing is performed only using a B-image signal (B narrow band data) mainly including information on the surface layer tissue and obtained by an imaging sensor after emitting the B narrow band light and a G-image signal (G narrow band data) mainly including information on the intermediate layer tissue and the surface layer tissue and obtained by an imaging sensor after emitting the G narrow band light, and an observation is performed by displaying a quasi-color image on a monitor or the like.

Therefore, in the image processing, the G-image signal (G narrow band data) obtained by the imaging sensor is allocated to R-image data of a color image through a predetermined coefficient, the B-image signal (B narrow band data) is allocated to G-image data and B-image data of a color image through a predetermined coefficient, a quasi-color image including 3-ch (channel) color image data is created, and the image is displayed on a monitor or the like.

For this reason, the image processing of the narrow band light mode converting two GB-image signals obtained by receiving the returned light of the narrow band light using the imaging sensor into RGB color image data for a quasi-color display on a display unit is different from the image processing of the ordinary light mode converting three RGB-image signals obtained by receiving the returned light of the ordinary light using the imaging sensor into RGB color image data for a color display on a display unit.

Further, even in the special light observation using the R narrow band light, the G narrow band light, and the B narrow band light, when the microstructure or the microscopic blood vessel of the surface layer tissue is observed, as described above, the image processing is performed only by using the G-image signal and the B-image signal without using the R-image signal (R narrow band data), and an observation is performed by displaying the quasi-color image on the monitor or the like.

Even in this case, in the image processing, in the same manner, the G-image signal is allocated to the R-image data, and the B-image signal is allocated to the G-image data and the B-image data, the quasi-color image including 3-ch color data is created, and the image is displayed on the monitor or the like.

As a result, in any case, since the quasi-color image displayed on the monitor or the like mainly includes the B-image signal (B narrow band data) including information on the surface layer tissue, the microstructure or the microscopic blood vessel of the surface layer tissue may be displayed in more detail, and the microstructure and the microscopic blood vessel of the surface layer tissue may be easily observed (refer to JP 3559755 B and JP 3607857 B).

In the special light observation described above, when the distance between the lesion tissue and the special light irradiation position is small, the microstructure or the microscopic blood vessel of the surface layer tissue, which may be easily brightly seen, may be displayed as an image, but there is a problem in that it is difficult to see the microstructure or the microscopic blood vessel of the surface layer tissue as the distance becomes larger.

Further, as described above, when the pixel size of the blood vessel projected to the imaging element changes due to a change in distance between the lesion tissue and the special light irradiation position and a change in magnification of the subject tissue, there is a problem in that it is difficult to recognize the microscopic blood vessel of the surface layer.

Furthermore, when the imaging position becomes farther away, each lump, that is, a region called a brownish region formed by densely aggregating surface layer microscopic blood vessels becomes an observation subject instead of each surface layer microscopic blood vessel, and although the image processing to be applied to the captured image is different, such a switching operation of the image processing is generally performed manually and an appropriate image emphasis is not reliably performed.

EP 1 980 199 A2 discloses an endoscope device in accordance with the preamble of claim 1. The image information detecting section may detect the subject information such as the hardness of a target within a body cavity of a subject. Brillouin scattering is used for measuring the hardness. In order to detect the scattering a two-wavelength laser is used and while a short wavelength beam is directed to the target, the reflected short wavelength beam is combined with a long wavelength laser beam so as to detect a frequency shift which is a measure of the hardness.

EP 2 179 687 A2 discloses an endoscope device in which an imaging condition such as imaging magnification, zoom magnification, amount of light emitted to the target, etc. is determined in dependency of whether an image to be obtained is obtained through near view imaging or distant view imaging.

WO 2009/128391 A1 discloses an illumination device used in the field of a dentistry. A first light source section emits white light to a broad area of teeth of a subject, while a second light source emits narrow band light to a small area in order to obtain an image in which the small area illuminated by the second light source has a hue which can be easily distinguished from the hue of the area which is only illuminated by the white light. In a specific embodiment (figure 25) an image pickup unit has a zoom lens and information about the magnification is used for changing the directional characteristic of the white light source.

### SUMMARY OF THE INVENTION

It is an object of the invention to provide an endoscope device capable of obtaining a bright captured image optimal for observing a structure and a component of a living body such as a microscopic blood vessel of a surface layer without making an operator intentionally adjust contents of image processing and a light emission amount such as a light emission ratio between white illumination light and special light while observing a captured image in a special light observation.

In order to solve the above-described problems, according to the present invention, there is provided an endoscope device comprising: a first light source section that emits narrow band light having a wavelength bandwidth narrowed in accordance with spectral characteristics of spectrums of a structure and a component of a living body as a subject; a second light source section that emits wide band light having a wide wavelength bandwidth including a visible region; an imaging section that captures an image of the subject using light returned from the living body after the narrow band light and the wide band light are simultaneously emitted from the first light source section and the second light source section to the subject, and outputs captured image information; an image processing section that performs a predetermined image processing on the captured image information; and an imaging information detecting section that detects as imaging information an automatic exposure value or an imaging magnification for capturing the subject using the imaging section, or subject information related to a structure and a component of the living body of the subject captured by the imaging section, wherein the narrow band light emitted from the first light source section has excellent detectability for the structure and the component of the living body of the subject compared to the wide band light emitted from the second light source section, and wherein light emission conditions of the first light source section and the second light source section and an image processing condition of the image processing section are changed so as to change detecting and emphasizing degrees of the structure and the component of the living body of the subject based on the imaging information detected by the imaging information detecting section.

In this case, it is preferable that the endoscope device further comprise: a light emission ratio changing section that changes light emission ratios of the narrow band light emitted from the first light source section and the wide band light emitted from the second light source section in order to change the light emission conditions of the first light source section and the second light source section.

In addition, it is preferable that the imaging information be the automatic exposure value, and the light emission ratio changing section increase a light emission ratio of the narrow band light emitted from the first light source section when the automatic exposure value is small, and increase a light emission ratio of the wide band light emitted from the second light source section when the automatic exposure value is large.

In addition, it is preferable that the imaging information be the imaging magnification, and the light emission ratio changing section increase a light emission ratio of the narrow band light emitted from the first light source section when the imaging magnification be large, and increase a light emission ratio of the wide band light emitted from the second light source section when the imaging magnification be small.

In addition, it is preferable that, when the light emission ratios be changed by the light emission ratio changing section, at least one of an electrical gain of the imaging section, an imaging time, and a color tone adjustment of the imaging processing be changed based on the light emission ratios such that a white balance of the captured image be not changed.

In addition, it is preferable that, when the light emission ratios be changed by the light emission ratio changing section, at least one of an electrical gain of the imaging section, an imaging time, and a color tone adjustment of the imaging processing be changed based on the light emission ratios such that a brightness of the captured image be not changed.

In addition, it is preferable that the image processing section include an image emphasizing section that change a frequency emphasis characteristic of the captured image based on the imaging information.

In addition, it is preferable that the image emphasizing section include a frequency band emphasizing section that emphasize two or more frequency bands of the captured image, and the frequency band emphasizing section change the frequency emphasis characteristic including a change in a frequency band to be emphasized based on the imaging information.

In addition, it is preferable that the imaging information be the automatic exposure value, and the frequency band emphasizing section change the frequency band to be emphasized to a low frequency side in accordance with an increase in the automatic exposure value.

In addition, it is preferable that the imaging information be the automatic exposure value, the frequency band emphasized by the frequency band emphasizing section be a band pass characteristic, and the frequency band emphasizing section change the frequency band to be emphasized so as to increase a width of the frequency band to be emphasized when the automatic exposure value exceed a first predetermined value.

In addition, it is preferable that the imaging information be the automatic exposure value, and the frequency band emphasizing section allow the frequency band to be emphasized to have a band pass characteristic when the automatic exposure value be a second predetermined value or less, and change the frequency band to be emphasized to have a high pass characteristic when the automatic exposure value exceed the second predetermined value.

In addition, it is preferable that the imaging information be the imaging magnification, the frequency band emphasizing section change the frequency band to be emphasized to a high frequency side in accordance with an increase in the imaging magnification.

In addition, it is preferable that the imaging information be the subject information related to a size of a brownish region or a thickness of a blood vessel, and the image emphasizing section change the frequency emphasis characteristic of the captured image based on the size of the brownish region or the thickness of the blood vessel.

In addition, it is preferable that the image emphasizing section include a frequency band emphasizing section that emphasizes two or more frequency bands of the captured image, and the frequency band emphasizing section change the frequency emphasis characteristic including a change in a frequency band to be emphasized based on the size of the brownish region or the thickness of the blood vessel.

In addition, it is preferable that the frequency band emphasizing section change the frequency band to be emphasized to a high frequency side in accordance with a decrease in the thickness of the blood vessel.

In addition, it is preferable that the frequency band emphasizing section allow the frequency band to be emphasized to have a band pass characteristic when the size of the brownish region be a predetermined size or less, and change the frequency band to be emphasized so as to increase a width of the frequency band to be emphasized when the size of the brownish region exceed the predetermined size.

In addition, it is preferable that the imaging information detecting section detect the imaging information from the captured image.

In addition, it is preferable that the imaging information detecting section detect the automatic exposure value from a brightness of the captured image.

According to the endoscope device of the invention, in the special light observation, the subject information related to the structure and the component of the captured living body or the automatic exposure value or the imaging magnification necessary for capturing the living body as the subject is detected as the imaging information, and the light emission conditions of the white illumination light source and the special light source and the image processing condition of the captured image are changed in order to change the detecting and emphasizing degrees of the structure and the component of the living body on the basis of the detected imaging information. Accordingly, in the case of performing the special light observation, for example, when a lesion is magnified or captured at a near position and the surface layer microscopic blood vessel is observed and when a lesion is captured at a far position and a brownish region having surface layer microscopic blood vessels densely aggregated therein is observed, the operator does not need to intentionally adjust or change the light emission condition of such a light source and the image processing condition of the captured image while observing the captured image, and a bright captured image optimal for the special light observation of the lesion or the surface layer microscopic blood vessel may be obtained.

### BRIEF DESCRIPTION OF THE DRAWINGS

Fig. 1 is a block diagram schematically illustrating an example of an entire configuration of an endoscope device of an embodiment of the invention.
Fig. 2 is a graph illustrating emission spectrums of narrow band light emitted from a narrow band laser beam source and quasi-white light emitted from a white light source including a blue laser beam source and a fluorescent body used for a light source unit of the endoscope device shown in Fig. 1.
Fig. 3 is a block diagram illustrating a signal processing system for respective sections including a specific configuration of an example of a processor of the endoscope device shown in Fig. 1.
Fig. 4 is a graph illustrating an example of a table defining a relation between a laser (LD) beam amount ratio and an automatic exposure (AE) value included in a necessary light amount ratio calculating section shown in Fig. 3.
Fig. 5 is a graph illustrating an example of a frequency emphasizing filter included in a structure emphasizing section of a special light image processing section shown in Fig. 3.
Fig. 6 is a flowchart illustrating a flow of an example of a narrow band light observation performed by the endoscope device shown in Fig. 1.

### DETAILED DESCRIPTION OF THE INVENTION

Hereinafter, an endoscope device according to the invention will be described in detail through a preferred embodiment shown in the accompanying drawings.

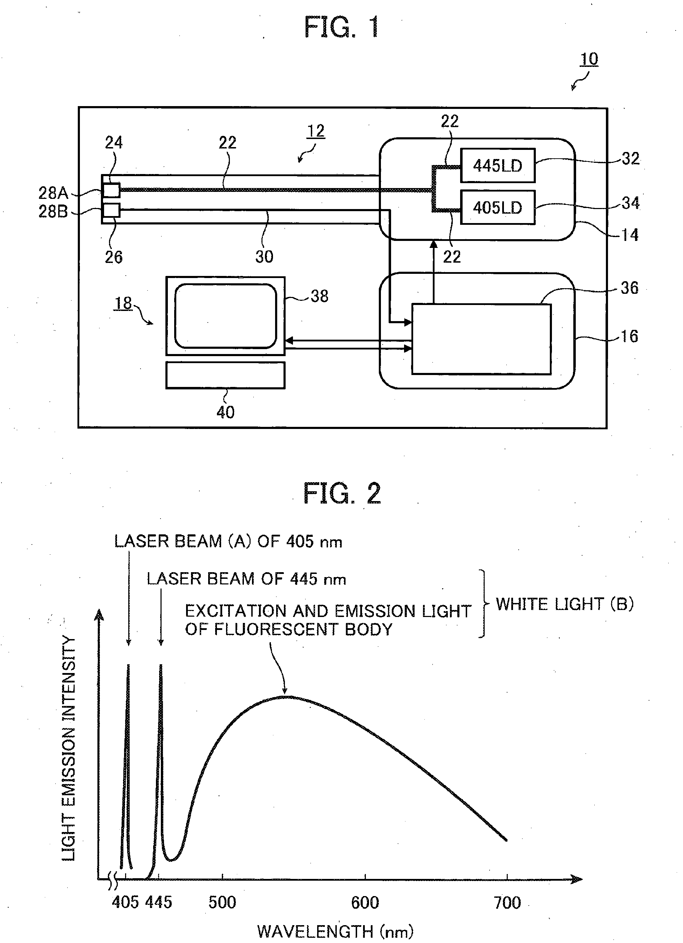

Fig. 1 is a block diagram schematically illustrating an example of an entire configuration of the endoscope device of the embodiment of the invention.

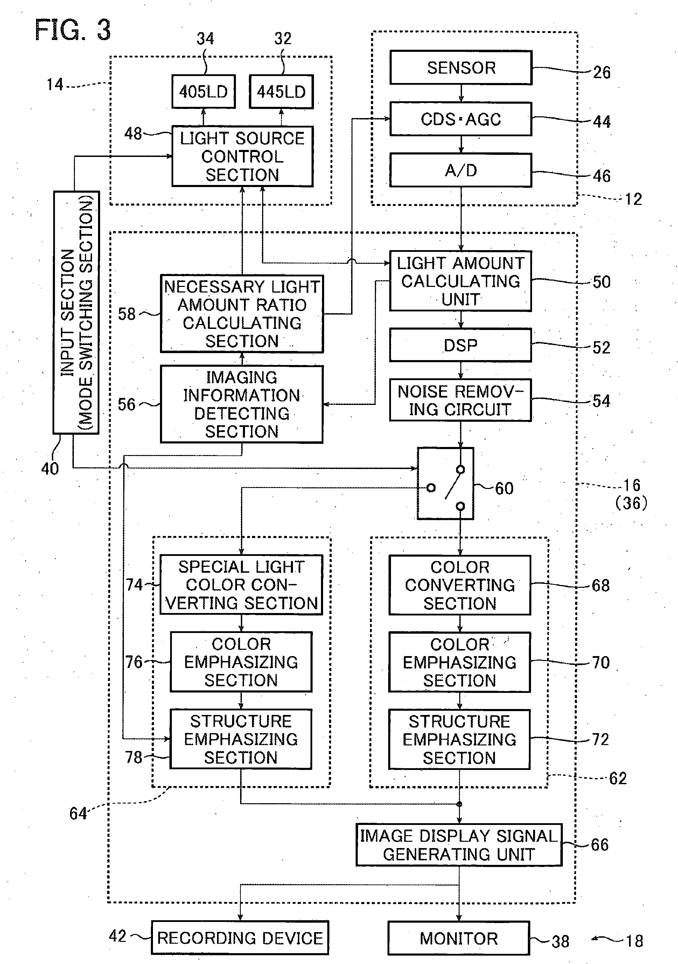

As shown in the same drawing, an endoscope device 10 of the invention includes an endoscope 12, a light source unit 14, a processor 16, and an input and output unit 18. Here, the light source unit 14 and the processor 16 constitute a control device of the endoscope 12, and the endoscope 12 is optically connected to the light source unit 14 and is electrically connected to the processor 16. Further, the processor 16 is electrically connected to the input and output unit 18. Then, the input and output unit 18 includes a display section (monitor) 38 that outputs and displays image information or the like, a recording section (recording device) 42 (refer to Fig. 3) that outputs image information or the like, and an input section (mode switching section) 40 that serves as a UI (user interface) receiving an input operation of function setting or mode switching for an ordinary observation mode (referred to as an ordinary light mode) or a special light observation mode (referred to as a special light mode).

The endoscope 12 is an electronic endoscope that includes an illumination optical system emitting illumination light from the front end thereof and an imaging optical system capturing an image of a subject observation region. Furthermore, although not shown in the drawings, the endoscope 12 includes an endoscope insertion section that is inserted into a subject, an operation section that is used to curve the front end of the endoscope insertion section or perform an observation, and a connector that attachably and detachably connects the endoscope 12 to the light source unit 14 and the processor 16 of the control device. Furthermore, although not shown in the drawings, the operation section and the endoscope insertion section are provided with various channels such as a clamp channel through which a tissue extracting treatment tool or the like is inserted or air and water supply channels.

As shown in Fig. 1, the front end of the endoscope 12 is provided with an irradiation port 28A that emits light to a subject observation region. Although it will be specifically described later, the irradiation port 28A is provided with an imaging element (sensor) 26 such as a CCD (Charge Coupled Device) image sensor or a CMOS (Complementary Metal-Oxide Semiconductor) image sensor that constitutes an illumination optical system, includes a fluorescent body 24 constituting a white light source, and acquires image information of the subject observation region at a light receiving portion 28B adjacent to the irradiation port 28A. The irradiation port 28A of the endoscope 12 is provided with a cover glass or a lens (not shown) constituting an irradiation optical system, the light receiving portion 28B is provided with a cover glass or a lens (not shown) constituting an illumination optical system, and a light receiving surface of the imaging element 26 of the light receiving portion 28B is provided with an objective lens (not shown) constituting an imaging optical system.

Further, the objective lens unit includes an objective lens (not shown). The field angle (viewing angle) of the objective lens is obtained according to the dimensions and the focal distance of the lens. The captured image formed by the imaging optical system becomes larger when the front end of the endoscope 12 becomes closer to the subject, and vice versa. Accordingly, an imaging magnification as a magnification between the subject and the capture image when capturing the image of the subject may be obtained from the field angle of the captured image.

The imaging magnification may be obtained in this manner. However, the method of obtaining the imaging magnification is not limited thereto, and various methods may be used.

For example, as disclosed in JP 2000-230807 A, the imaging magnification may be automatically detected in a manner such that light parallel to an optical axis of an imaging optical system is emitted to a subject using a laser or the like from an illumination optical system and a length for an imaging viewing field of an image formed by the imaging optical system by the returned light is measured.

Furthermore, the objective lens unit may include a high magnification imaging mechanism including an imaging lens (not shown) movable in the direction of an optical axis and a lens driving mechanism (not shown) moving the imaging lens in order to change the imaging magnification. In this case, the lens driving mechanism includes, for example, an actuator configured as a piezoelectric element, and may further change the imaging magnification by moving the imaging lens in the direction of the optical axis.

The endoscope insertion section may be freely curved by the operation of the operation section, may be curved at an arbitrary angle in an arbitrary direction in accordance with a portion or the like of the subject where the endoscope 12 is used, and may direct the observation direction of the irradiation port 28A and the light receiving portion 28B, that is, the imaging element 26 to a desired observation portion.

Furthermore, it is desirable that the imaging element 26 be a complementary color sensor or an imaging sensor including a color filter (for example, an RGB color filter or a complementary color filter) in the light receiving region, but it is more desirable to use an RGB color image sensor.

The light source unit 14 includes a light source, that is, a blue laser beam source (445LD) 32 having a central wavelength of 445 nm and used as a white illumination light source used for both an ordinary light mode and a special light mode and a blue-violet laser beam source (405LD) 34 having a central wavelength of 405 nm and used as a special light source in a special light mode. Furthermore, the blue-violet laser beam having a central wavelength of 405 nm output from the blue-violet laser beam source 34 has an excellent detecting property for a structure and a component of a living body since it is narrow band light having a wavelength bandwidth narrowed in accordance with the emission spectrum of the structure and the component of the living body.

The light emission from the semiconductor light emitting elements of the light sources 32 and 34 is individually controlled by a light source control section 48 (refer to Fig. 3), and the light emission conditions of each of the light sources 32 and 34, that is, the light amount ratio (light emission ratio) between the light emitted from the blue laser beam source 32 and the light emitted from the blue-violet laser beam source 34 may be freely changed.

As the blue laser beam source 32 and the blue-violet laser beam source 34, a broad area type InGaN laser diode, an InGaNAs laser diode, or a GaNAs laser diode may be used. Further, the light source may be configured as a light emitter such as a light emitting diode.

The laser beams emitted from the light sources 32 and 34 are respectively input to optical fibers 22 by a condensing lens (not shown), and are transmitted to the connector through a multiplexer (not shown). Furthermore, the invention is not limited thereto, and a configuration may be adopted in which the laser beams output from the light sources 32 and 34 are directly transmitted to the connector without using the multiplexer.

The laser beam, which is obtained by multiplexing the blue laser beam having a central wavelength of 445 nm and the blue-violet laser beam having a central wavelength of 405 nm and is transmitted to the connector, is propagated to the front end of the endoscope 12 by the optical fiber 22 constituting the illumination optical system. Then, the blue laser beam emits fluorescence by exciting the fluorescent body 24 as a wavelength converting member disposed at the light emission end of the optical fiber 22 of the front end of the endoscope 12. Further, a part of the blue laser beam is directly transmitted through the fluorescent body 24. The blue-violet laser beam is transmitted through the fluorescent body 24 without any excitation, so that it becomes illumination light of a narrow band wavelength (so-called narrow band light).

The optical fiber 22 is a multi-mode fiber, and an example thereof includes a thin fiber cable having a core diameter of 105 µm, a cladding diameter of 125 µm, and a diameter, including a protective layer as an outer coat, of φ0.3 to 0.5 mm.

The fluorescent body 24 includes a plurality of types of fluorescent bodies (for example, a YAG-based fluorescent body or a fluorescent body of BAM (BaMgAl₁₀O₁₇) or the like) absorbing a part of the blue laser beam and emitting green to yellow light by being excited. Accordingly, white (quasi-white) illumination light is obtained by combining green to yellow excitation light using the blue laser beam as excitation light and the blue laser beam transmitted through the fluorescent body 24 without being absorbed thereto. As in the configuration example, when the semiconductor light emitting element is used as an excited light source, it is possible to obtain high-intensity white light with high light emitting efficiency, easily adjust the intensity of white light, and suppress a change in the color temperature and chromaticity of the white light as small as possible.

The fluorescent body 24 may prevent flickering generated when displaying a dynamic image, or noise overlapping disturbing an imaging operation due to speckles generated by coherence of a laser beam. Further, it is desirable that the fluorescent body 24 be formed in consideration of a difference in refractive index between a fluorescent material constituting the fluorescent body and a fixing and solidifying resin forming a filling material. The particle diameter of the material of the fluorescent material and the filling material preferably has small absorption and great scattering with respect to light of an infrared region. Accordingly, it is possible to improve a scattering effect without degrading light intensity with respect to light of a red or infrared region, and reduce optical loss.

Fig. 2 is a graph illustrating emission spectrums of the blue-violet laser beam output from the blue-violet laser beam source 34, the blue laser beam output from the blue laser beam source 32, and the light obtained by converting the wavelength of the blue laser beam through the fluorescent body 24. The blue-violet laser beam is depicted by the emission line (profile A) having a central wavelength of 405 nm, is the narrow band light of the invention, and is used as special light. Further, the blue laser beam is depicted by the emission line having a central wavelength of 445 nm. The excitation and emission light obtained from the fluorescent body 24 using the blue laser beam substantially has a wavelength bandwidth of 450 nm to 700 nm, and has a spectral intensity distribution in which light emission intensity increases. By the profile B formed by the excitation and emission light and the blue laser beam, the above-described quasi-white light is formed, and is used as ordinary light.

Here, the white light mentioned in the invention is not precisely limited to the light including all wavelength components of the visible light, but may include, for example, light of a specific wavelength such as R, G, and B including the above-described quasi-white light. In a broad sense, for example, light including green to red wavelength components or light including blue to green wavelength components is included.

In the endoscope device 10, the light emission intensities of the profile A and the profile B are controlled to be relatively increased and decreased by the light source control section 48, so that an illumination port with an arbitrary luminance balance may be generated. Furthermore, in the endoscope device 10 of the invention, only the light of the profile B is used in the ordinary light mode, and the light obtained by overlapping the profiles A and B with each other is used in the special light mode.

As described above, the white light (profile B) obtained by the excitation and emission light from the fluorescent body 24 and the blue laser beam from the blue laser beam source (hereinafter, referred to as 445LD) 32 and the illumination light (profile A) including the narrow band light formed by the blue-violet laser beam from the blue-violet laser beam source (hereinafter, referred to as 405LD) 34 are emitted from the irradiation port 28A of the front end of the endoscope 12 to the subject observation region. Then, the light returned from the subject observation region after emitting the illumination light thereto is formed on the light receiving surface of the imaging element 26 through the light receiving portion 28B, and the subject observation region is captured by the imaging element 26.

The image signal of the captured image output from the imaging element 26 after the imaging operation is input to an image processing system 36 of the processor 16 through a scope cable 30.

Next, the image signal of the image captured by the imaging element 26 in this manner is processed by the signal processing system including the image processing system 36 of the processor 16, is output to a monitor 38 or a recording device 42, and is provided for observation by the user.

Fig. 3 is a block diagram illustrating the signal processing system for respective sections including a specific configuration of an example of the processor of the endoscope device of the invention.

As shown in the same drawing, the signal processing system of the endoscope device 10 includes the signal processing system of the endoscope 12, the signal processing system of the light source unit 14, the signal processing system (image processing system 36) of the processor 16, the monitor 38 of the input and output unit 18, the input section (mode switching section) 40, and the recording device 42.

The signal processing system of the endoscope 12 is a signal processing system of an image signal of a captured image from the imaging element 26 after the imaging operation, and includes a CDS and AGC circuit 44 that performs a correlated double sampling (CDS) or an automatic gain control (AGC) on a captured image signal as an analog signal and an A/D converter 46 that converts the analog image signal subjected to the sampling and the gain control in the CDS and AGC circuit 44 into a digital image signal. The digital image signal A/D converted in the A/D converter 46 is input to the image processing system 36 of the processor 16 through the connector.

Further, the signal processing system of the light source unit 14 includes the light source control section 48 that performs a light amount control and an on/off control of the blue laser beam source (445LD) 32 and the blue-violet laser beam source (405LD) 34.

Here, the light source control section 48 turns the blue laser beam source 32 on in accordance with a light source on signal with the activation of the endoscope device 10, performs on and off control of the blue-violet laser beam source 34 in accordance with the switching signal between the ordinary light mode and the special light mode from the mode switching section 40, or controls the light emission intensities of the blue laser beam source 32 and the blue-violet laser beam source 34, that is, the current value flowing to the light sources 32 and 34 in accordance with the light amount of the B light and the G light of the image calculated from the light amount calculating unit 50 to be described later or the light emission intensities of the profiles A and B. That is, the light source control section 48 serves as a light emission ratio changing section that changes the light emission conditions, that is, the light emission ratio between both light sources 32 and 34 on the basis of the imaging information such as the automatic exposure (AE) value (light amount ratio) and the imaging magnification detected in an imaging information detecting section 56 or subject information related to the structure and the component of the living body such as the thickness of the blood vessel or the size of the brownish region together with a necessary light amount ratio calculating section 58 to be described later.

Furthermore, the signal processing system of the processor 16 is the image processing system 36 (refer to Fig. 1), and includes the light amount calculating unit 50, a DSP (digital signal processor) 52, a noise removing circuit 54, an image processing switching section (switch) 60, an ordinary light image processing unit 62, a special light image processing unit 64, and an image display signal generating unit 66.

The light amount calculating unit 50 uses the digital image signal input from the A/D converter 46 of the endoscope 12 through the connector, and calculates the light amount of the returned light received at the imaging element 26, for example, the light amounts of the B light and the G light, that is, the light amount of the B light and the G light of the image. Then, the light amount calculating unit 50 calculates the light amount ratio (B/G ratio) of the B light and the G light of the captured image on the basis of the light amounts of the B light and the G light of the calculated image.

Further, the light amount calculating unit 50 calculates the light source light amount, that is, the light amount (light emission intensity) of the blue laser beam from the 445LD 32, the light amount (the light emission intensity of the profile B shown in Fig. 2) of the quasi-white light from the fluorescent body 24 using the blue laser beam, the light amount (the light emission intensity of the profile A shown in Fig. 2) of the blue-violet laser beam of the 405LD 34, or the like, and obtains the light amount ratio (the light emission ratio of 405LD/445LD) between the 445LD 32 and the 405LD 34 on the basis of these.

Then, the light amount calculating unit 50 calculates the brightness (luminance value) of the captured image on the basis of the RGB value of the calculated captured image, and outputs the result to the imaging information detecting section 56 together with the light amount and the light amount ratio (the light emission ratio of 405LD/445LD) of the 445LD 32 and the 405LD 34.

The imaging information detecting section 56 calculates the imaging information on the basis of the light amount and the light amount ratio (the light emission ratio) of the 445LD 32 and the 405LD 34. Here, as the imaging information, the automatic exposure (AE) value (light amount value) or the imaging magnification for imaging the subject (living body) or subject information related to the structure and the component of the living body such as the thickness of the blood vessel or the size of the brownish region may be exemplified.

Here, the automatic exposure value (AE value) indicates a parameter for automatically determining the exposure during the imaging operation, and is determined on the basis of the light amount (brightness) of the returned light detected by the imaging element 26. Even when shooting a video, the parameter is determined by the light amount of the returned light in the imaging time for each frame determined in accordance with the accumulated time (the accumulated time of the CCD or the CMOS corresponding to the RGB color filter) of the imaging element 26.

As described above, the imaging magnification may be obtained from the field angle of the captured image, and generally automatically detected as described above. Furthermore, when the imaging optical system includes a high-magnification imaging mechanism, the imaging magnification is changed in accordance with a distance between the objective lens and the imaging lens.

Further, the subject information indicates information related to the structure and the component of the living body such as the thickness or the like of each blood vessel in the magnification imaging operation or the near-distance imaging operation or the size of the brownish region, that is, the region where the surface layer microscopic blood vessels of a lesion are aggregated in a far-distance imaging operation. The size of the brownish region or the thickness of the blood vessel is detected by extracting the brownish region from the captured image or extracting each blood vessel. The brownish region may be extracted by using various known methods of detecting the color or the shape. In the invention, it is desirable to change the image processing applied to the captured image when the thickness of the blood vessel or the size of the brownish region detected in the captured image changes.

When such imaging information is detected, the information is output to the necessary light amount ratio calculating section 58 and the special light image processing unit 64 to be described later.

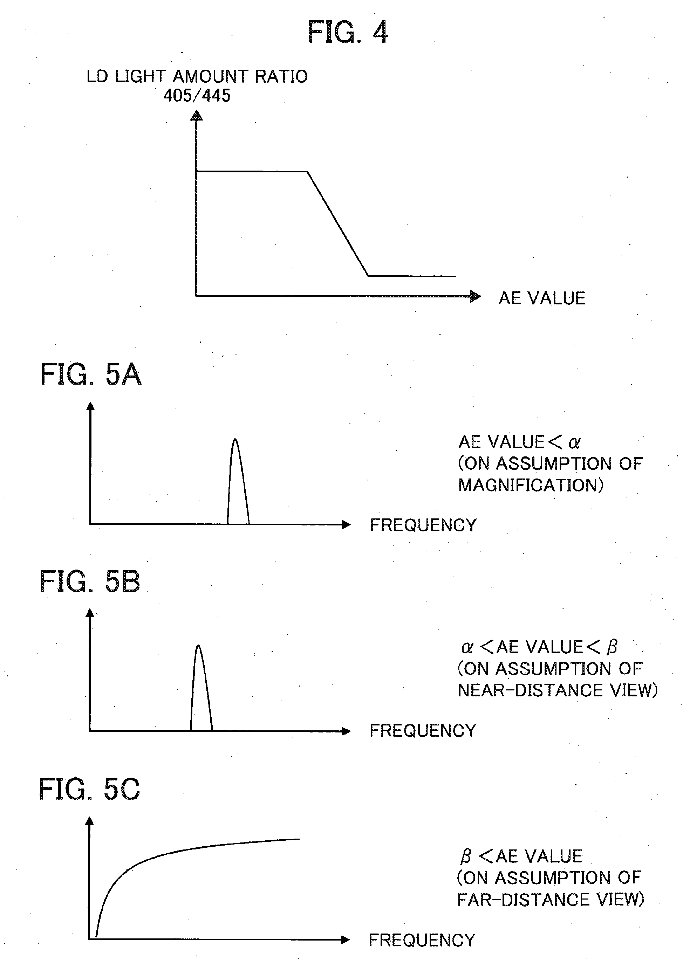

The necessary light amount ratio calculating section 58 calculates the light amount ratio and the light amount necessary for the imaging operation on the basis of the detected imaging information in the imaging information detecting section 56. For example, as shown in Fig. 4, the necessary light amount ratio calculating section 58 includes a table representing a relation between the AE value and the LD light amount ratio of 405LD/445LD, calculates the 405LD/445LD light amount ratio on the basis of the AE value as the imaging information and the table, and further calculates the light amounts of the 405LD and the 445LD.

The light amount and the light amount ratio of the 405LD and the 445LD are output to the light source control section 48.

Furthermore, since the white balance of the captured image changes in accordance with a change of the light amount ratio of the laser, the light amount and the light amount ratio of the 405LD and the 445LD are output to the CDS and AGC circuit 44. Then, the gain of the CDS and AGC circuit 44 obtaining the white balance on the basis of the information of the light amount and the light amount ratio also changes, so that the electrical gain of the imaging element 26 changes.

The DSP 52 (digital signal processor) performs a gamma correction process and a color correction process on the digital image signal output from the A/D converter 46 after detecting the light source light amount at the light amount calculating unit 50.

The noise removing circuit 54 removes noise from the digital image signal subjected to the gamma correction process and the color correction process in the DSP 52 by performing, for example, a noise removing method in the image processing such as a moving-average method or a median filtering method.

In this manner, the digital image signal input from the endoscope 12 to the processor 16 is subjected to a pre-process such as a gamma correction process, a color correction process, and a noise removing process at the DSP 52 and the noise removing circuit 54.

The image processing switching section 60 is a switch that switches the transmission destination of the digital image signal subjected to a pre-process to the special light image processing unit 64 or the ordinary light image processing unit 62 at the rear stage on the basis of the instruction (switching signal) of the mode switching section (input section) to be described later.

Furthermore, in the invention, to distinguish them, the digital image signal before the image processing using the ordinary light image processing unit 62 and the special light image processing unit 64 is referred to as an image signal, and the digital image signal before and after the image processing is referred to as image data.

The ordinary light image processing unit 62 is a unit that performs ordinary light image processing suitable for the digital image signal subjected to the pre-process using the white light (profile B) of the fluorescent body 26 and the 445LD in the ordinary light mode, and includes a color converting section 68, a color emphasizing section 70, and a structure emphasizing section 72.

The color converting section 68 performs a color conversion process such as a three-dimensional LUT process, a grayscale conversion process, and a three by three matrix process on the digital image signals of RGB three channels subjected to the pre-process, so that it is converted into RGB image data subjected to the color conversion process.

The color emphasizing section 70 is used to emphasize the blood vessel so as to be easily viewed by showing a difference in hue between the blood vessel and the mucous in the screen, and performs a process on the RGB image data subjected to the color conversion process while seeing the screen. The process is, for example, a process that emphasizes a difference in hue between the blood vessel and the mucous from the average value while seeing the average hue of the entire screen.

The structure emphasizing section 72 performs a structure emphasizing process such as a sharpening process or an outline emphasizing process on the RGB image data subjected to the color emphasizing process.

The RGB image data subjected to the structure emphasizing process in the structure emphasizing section 72 is input as the RGB image data subjected to the ordinary light image processing from the ordinary light image processing unit 62 to the image display signal generating unit 66.

The special light image processing unit 64 is a unit that performs special light image processing suitable for the digital image signal subjected to the pre-process using the white light (profile B) from the fluorescent body 26, the 445LD 32, and the blue-violet laser beam (profile A) from the 405LD 34 in the special light mode, and includes a especial light color converting section 74, a color emphasizing section 76, and a structure emphasizing section 78.

The special light color converting section 74 allocates the G-image signal of the digital image signals of the RGB three channels subjected to the pre-process to the R-image data through a predetermined coefficient, and allocates the B-image signal to the G-image data and B-image data through a predetermined coefficient so as to generate the RGB image data. Then, the generated RGB image data is subjected to a color conversion process such as a three-dimensional LUT process, a grayscale conversion process, and a three by three matrix process as in the color converting section 68.

As in the color emphasizing section 70, the color emphasizing section 76 is used to emphasize the blood vessel so as to be easily viewed by showing a difference in hue between the blood vessel and the mucous in the screen, and performs a process on the RGB image data subjected to the color conversion process while seeing the screen. The process is, for example, a process that emphasizes a difference in hue between the blood vessel and the mucous from the average value while seeing the average hue of the entire screen.

The structure emphasizing section 78 performs a structure process such as a sharpening process or an outline emphasizing process on the RGB image data subjected to the color emphasizing process as in the structure emphasizing section 72.

Further, in addition to the structure process of the structure emphasizing section 72, the structure emphasizing section 78 performs a frequency emphasizing process on the RGB image data subjected to the above-described color emphasizing process on the basis of the imaging information from the imaging information detecting section 56, for example, the AE value.

As shown in Figs. 5A to 5C, the frequency emphasizing process performed herein is different in accordance with the AE value. Here, a case is described in which the AE value is used as a representative example of the imaging information, but it is needless to mention that the invention is not limited thereto.

When the AE value is smaller than the first predetermined value (α), that is, a magnification observation is assumed in which the front end of the endoscope becomes closer to the subject and a small necessary light amount is needed, the surface layer microscopic blood vessel is assumed as the imaging subject, and the frequency emphasizing filter capable of emphasizing the high frequency part as shown in Fig. 5A is applied to the above-described RGB image data so that the microstructure of the surface layer microscopic blood vessel may be divided into thin lines.

Further, when the AE value is in a predetermined range (a range from α to β) between the first predetermined value and the second predetermined value, that is, a near-distance observation is assumed in which the front end of the endoscope is slightly distant from the subject and a light amount slightly larger than the magnification observation is needed, each microscopic blood vessel slightly larger than the imaging subject as the microstructure of the surface layer microscopic blood vessel is assumed as an imaging subject, and the frequency emphasizing filter capable of emphasizing the middle frequency part as shown in Fig. 5B is applied to the above-described RGB image data so that the ambient part of the surface layer microscopic blood vessel is emphasized.

Furthermore, when the AE value is larger than the second predetermined value (β), that is, a far-distance observation is assumed in which the front end of the endoscope becomes farther from the subject and the larger light amount is needed, a brownish region formed by aggregating the surface layer microscopic blood vessels and present as a lump is assumed as the imaging subject instead of a single surface layer microscopic blood vessel.

The region called the brownish region is assumed to be an early cancer, and in many cases, the size thereof is 1 mm or so, but the size thereof may be 2 mm or 3 mm. When the filter with the band pass characteristic is used in order to emphasize the frequency band, the emphasis is not performed when slightly deviating from the band of the band pass. For this reason, it is desirable to use a filter with a high pass characteristic in order to emphasize all brownish regions with various sizes.

Accordingly, when the brownish region is assumed as the imaging subject, it is desirable to use the high pass filter capable of emphasizing the entire high frequency as shown in Fig. 5C as the frequency emphasizing filter and applies the filter to the above-described RGB image data.

The RGB image data subjected to the optimal frequency emphasizing process on the basis of the AE value in the structure emphasizing section 72 is input as the RGB image data subjected to the special light image processing from the special light image processing unit 64 to the image display signal generating unit 66.

The image display signal generating unit 66 converts the RGB image data subjected to the image processing input from the ordinary light image processing unit 62 in the ordinary light mode and the RGB image data subjected to the image processing input from the special light image processing unit 64 in the special light mode into a display image signal to be displayed as a soft copy image in the monitor 38 or a display image signal to be output as a hard copy image in the recording device 42.

In the ordinary light mode, the monitor 38 displays the ordinary observation image, which is based on the display image signal obtained in the imaging element 26 by emitting the white light and subjected to the pre-process and the ordinary light image processing in the processor 16, as a soft copy image, and, in the special light mode, displays the special light observation image, which is based on the display image signal obtained in the imaging element 26 by emitting the special light in addition to the white light and subjected to the pre-process and the special light image processing in the processor 16, as a soft copy image.

The recording device 42 also outputs the hard copy image, that is, the ordinary observation image obtained by emitting the white light in the ordinary light mode, and outputs the hard copy image, that is, the special light observation image obtained by emitting the white light and the special light in the special light mode.

Furthermore, if necessary, the display image signal generated in the image display signal generating unit 66 may be stored as image information in a storage unit including a memory or a storage device (not shown).

On the other hand, the mode switching section (input section) 40 includes a mode switching button that switches the ordinary light mode and the special light mode, and the mode switching signal from the mode switching section 40 is input to the light source control section 48 of the light source unit 14. Here, the mode switching section 40 is disposed as the input section 40 of the input and output unit 18, but may be disposed at the processor 16, the operation section of the endoscope 12, or the light source unit 14. Furthermore, the switching signal from the mode switching section 40 is output to the light source control section 48 and the image processing switching section 60.

The endoscope device of the invention basically has the above-described configuration.

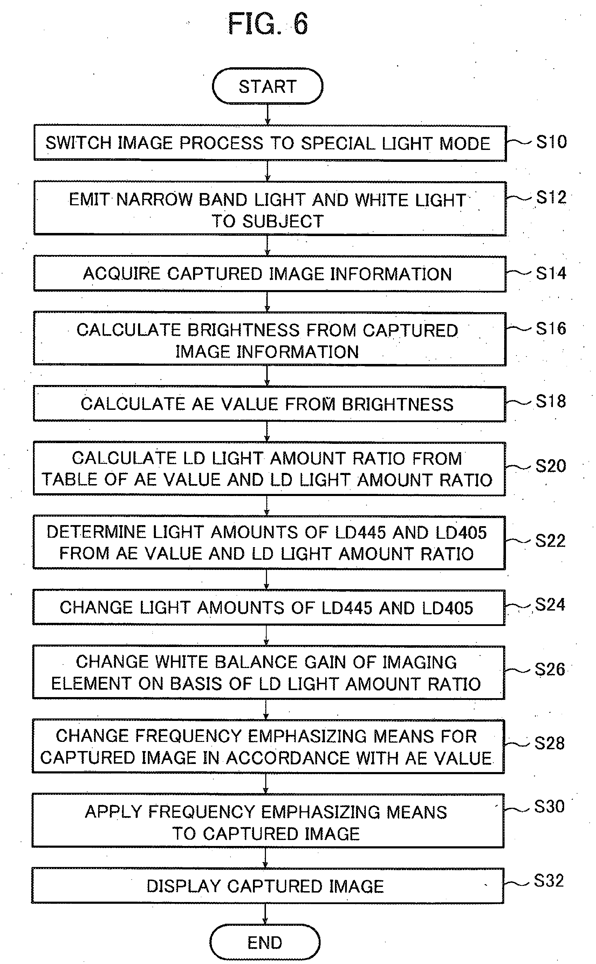

Hereinafter, an operation of the endoscope device of the invention will be described by referring to Fig. 6.

In the embodiment, first, it is assumed that the ordinary light observation is performed in the ordinary light mode. It is assumed that the 445LD 32 is turned on, and the ordinary light image processing is performed on the captured image data using the white light in the ordinary light image processing unit 64.

Here, the special light mode is switched by a user. When the user operates the mode switching section 40, a mode switching signal (special light ON) is output, and the image processing in the image processing switching section 60 is switched to the special light mode (S10).

Subsequently, the mode switching signal is also input to the light source control section 40 of the light source unit 14, the 405LD 34 is turned on by the light source control section 40, and the white light and the narrow band light are simultaneously emitted toward the subject (S12).

The white light and the narrow band light simultaneously emitted are reflected by the subject, and the captured image information is acquired by the imaging element 26 (S14).

Next, the captured image information acquired by the imaging element 26 is subjected to a white gain adjustment and is converted into digital data, and is transmitted to the light amount calculating unit. In the captured image information, the brightness (luminance value) of the captured image (RGB image) is calculated in the light amount calculating unit 50 (S16).

The information on the brightness (luminance value) of the RGB image calculated in the light amount calculating unit 50 is transmitted to the imaging information detecting section 56, and the AE value for an imaging operation is detected (S18).

Further, instead of the AE value, the imaging magnification of the imaging operation or information (the size of the brownish region, the thickness of the blood vessel, or the like) of the subject may be detected.

The detected AE value is output to the necessary light amount ratio calculating section 58 and the special light image processing unit 64.

The necessary light amount ratio calculating section 58 receives the calculated AE value, and calculates the necessary light amount ratio (S20). As shown in Fig. 4, the necessary light amount ratio calculating section 58 includes a table representing a relation between the AE value and the LD light amount ratio, and calculates the LD light amount ratio in accordance with the AE value.

The LD light amount ratio is a ratio between the light emission amounts of the 405LD 34 and the 445LD 32, and calculates the necessary light amount of each of the light amount of the 445LD 32 and the light amount of the 405LD 34 from the brightness (luminance value) of the captured image calculated in the light amount calculating unit 50 and the calculated LD light amount ratio (405LD/445LD) (S22). The calculated necessary light amount ratio is output to the CDS and AGC circuit 44 in order to adjust the white balance gain, and the calculated necessary light amount ratio is output to the light source control section 48.

The light source control section 48 performs a control so that the light emission amounts from the 445LD 32 and the 405LD 34 become the necessary light amount on the basis of the necessary light amounts of the 445LD 32 and the 405LD 34 (S24).

Further, the CDS and AGC circuit 44 adjusts a white balance gain on the basis of the calculated necessary light amount ratio (S26).

When the light emission amounts from the 445LD 32 and 405LD 34 change, the white balance gain changes in accordance with the change, so that the CDS and AGC is adjusted so that the white balance gain is maintained at a constant value. Further, the imaging time or the color tone adjustment of the image processing may be changed instead of the adjustment of the white balance gain of the CDS and AGC.

Further, the imaging information detecting section 56 changes the contents of the image processing for the captured image on the basis of the calculated AE value (S28). The image processing changed on the basis of the AE value is performed by the structure emphasizing section 80 of the special light image processing unit 64.

The captured image information obtained in the narrow band light observation is output to the special light image processing unit 64, the above-described image processing is performed through the special light color converting section 74 and the color emphasizing section 76, and the result is input to the structure emphasizing section 78. In the structure emphasizing section 78, as described above, the frequency emphasizing filter shown in Figs. 5A to 5C is applied in accordance with the AE value (S30).

In the special light image processing unit 64, the image information subjected to the image processing through the frequency emphasizing filter according to the AE value is output to the image display signal generating unit 66. The image display signal generating unit 66 generates and outputs an image display signal from the image information.

The output image display signal is displayed as a special light image on the monitor 38, and is recorded on the recording device 42 (S32).

While the endoscope device of the invention has been described in detail, the invention is not limited to the above-described embodiment, and various modifications or changes may be performed within the scope of the invention.

## Claims

1. An endoscope device (10) comprising:
a first light source section (34) that emits narrow band light having a -wavelength bandwidth narrowed in accordance with spectral characteristics of emission spectrums of a structure and a component of a living body as a subject, such as the thickness or the like of a blood vessel;
a second light source section (32, 24) that emits wide band light having a wide wavelength bandwidth wider than said narrowed bandwidth and including a visible region;
an imaging section (12, 26) that captures an image of said subject using light returned from said living body after said narrow band light and said wide band light are simultaneously emitted from said first light source section (34) and said second light source section (32) to said subject, and outputs captured image information;
an image processing section (36) that performs a predetermined image processing on said captured image information; and
an imaging information detecting section (56) **characterized in that** said imaging information detecting section detects as imaging information an automatic exposure value e.g. a light amount value, or an imaging magnification for capturing said subject using said imaging section (12, 26), or subject information related to a structure and a component of said living body of said subject captured by said imaging section,
wherein said narrow band light emitted from said first light source section (34) has excellent detectability for the structure and the component of said living body of said subject compared to said wide band light emitted from said second light source section (32, 24), and
wherein light emission conditions of said first light source section (34) and said second light source section (32, 24) and an image processing condition of said image processing section (36) are changed so as to change detecting and emphasizing degrees of the structure and the component of said living body of said subject based on said imaging information detected by said imaging information detecting section (56).

2. The endoscope device according to claim 1, further comprising:
a light emission ratio changing section that changes light emission ratios of said narrow band light emitted from said first light source section and said wide band light emitted from said second light source section in order to change said light emission conditions of said first light source section and said second light source section.

3. The endoscope device according to claim 2,
wherein said imaging information is said automatic exposure value, and
wherein said light emission ratio changing section increases a light emission ratio of said narrow band light emitted from said first light source section when said automatic exposure value is small, and increases a light emission ratio of said wide band light emitted from said second light source section when said automatic exposure value is large.

4. The endoscope device according to claim 2,
wherein said imaging information is said imaging magnification, and
wherein said light emission ratio changing section increases a light emission ratio of said narrow band light emitted from said first light source section when said imaging magnification is large, and increases a light emission ratio of said wide band light emitted from said second light source section when said imaging magnification is small.

5. The endoscope device according to any one of claims 2 to 4,
wherein, when said light emission ratios are changed by said light emission ratio changing section, at least one of an electrical gain of said imaging section, an imaging time, and a color tone adjustment of the imaging processing is changed based on said light emission ratios such that a white balance of said captured image is not changed.

6. The endoscope device according to any one of claims 2 to 5,
wherein, when said light emission ratios are changed by said light emission ratio changing section, at least one of an electrical gain of said imaging section, an imaging time, and a color tone adjustment of the imaging processing is changed based on said light emission ratios such that a brightness of said captured image is not changed.

7. The endoscope device according to any one of claims 1 to 6,
wherein said image processing section includes an image emphasizing section that changes a frequency emphasis characteristic of said captured image based on the imaging information.

8. The endoscope device according to claim 7,
wherein said image emphasizing section includes a frequency band emphasizing section that emphasizes two or more frequency bands of said captured image, and
wherein said frequency band emphasizing section changes said frequency emphasis characteristic including a change in a frequency band to be emphasized based on said imaging information.

9. The endoscope device according to claim 8,
wherein said imaging information is said automatic exposure value, and
wherein said frequency band emphasizing section changes said frequency band to be emphasized to a low frequency side in accordance with an increase in said automatic exposure value.

10. The endoscope device according to claim 8 or 9,
wherein said imaging information is said automatic exposure value,
wherein said frequency band emphasized by said frequency band emphasizing section is a band pass characteristic, and
wherein said frequency band emphasizing section changes said frequency band to be emphasized so as to increase a width of said frequency band to be emphasized when said automatic exposure value exceeds a first predetermined value.

11. The endoscope device according to any one of claims 8 to 10,
wherein said imaging information is said automatic exposure value, and
wherein said frequency band emphasizing section allows said frequency band to be emphasized to have a band pass characteristic when said automatic exposure value is a second predetermined value or less, and changes said frequency band to be emphasized to have a high pass characteristic when said automatic exposure value exceeds said second predetermined value.

12. The endoscope device according to claim 8,
wherein said imaging information is said imaging magnification,
wherein said frequency band emphasizing section changes said frequency band to be emphasized to a high frequency side in accordance with an increase in said imaging magnification.

13. The endoscope device according to claim 7,
wherein said imaging information is said subject information related to a size of a brownish region or a thickness of a blood vessel, and
wherein said image emphasizing section changes said frequency emphasis characteristic of said captured image based on the size of said brownish region or the thickness of said blood vessel.

14. The endoscope device according to claim 13,
wherein said image emphasizing section includes a frequency band emphasizing section that emphasizes two or more frequency bands of said captured image, and
wherein said frequency band emphasizing section changes said frequency emphasis characteristic including a change in a frequency band to be emphasized based on the size of the brownish region or the thickness of the blood vessel.

15. The endoscope device according to claim 14,
wherein said frequency band emphasizing section changes said frequency band to be emphasized to a high frequency side in accordance with a decrease in the thickness of said blood vessel.

16. The endoscope device according to claim 14,
wherein said frequency band emphasizing section allows said frequency band to be emphasized to have a band pass characteristic when the size of said brownish region is a predetermined size or less, and changes said.frequency band to be emphasized so as to increase a width of said frequency band to be emphasized when the size of said brownish region exceeds said predetermined size.

17. The endoscope device according to any one of claims 1 to 16,
wherein said imaging information detecting section detects said imaging information from said captured image.

18. The endoscope device according to claim 17,
wherein said imaging information detecting section detects said automatic exposure value from a brightness of said captured image.

## Patentansprüche

1. Endoskopvorrichtung (10), umfassend:
einen ersten Lichtquellenabschnitt (34), der schmalbandiges Licht mit einer Wellenlängen-Bandbreite emittiert, die verengt ist nach Maßgabe der Spektralkennlinie von Emissionsspektren einer Struktur und einer Komponente in einem lebenden Körper als Subjekt, so zum Beispiel der Dicke oder dergleichen eines Blutgefäßes;
einen zweiten Lichtquellenabschnitt (32, 24), der breitbandiges Licht mit einer breiten Wellenlänge-Bandbreite größer als jener der verengten Bandbreite und eine sichtbare Zone enthaltend emittiert;
einen Abbildungsabschnitt (12, 26), der ein Bild des Subjekts unter Verwendung von Licht aufnimmt, welches von dem lebenden Körper zurückgeworfen wird, nachdem von dem ersten Lichtquellenabschnitt (34) und dem zweiten Lichtquellenabschnitt (32) das schmalbandige Licht und das breitbandige Licht gleichzeitig auf das Subjekt emittiert wurden, und der aufgenommene Bildinformation ausgibt;
einen Bildverarbeitungsabschnitt (36), der eine vorbestimmte Bildverarbeitung an der aufgenommenen Bildinformation vornimmt; und
einen Abbildungsinformations-Nachweisabschnitt (56), **dadurch gekennzeichnet, dass** der Abbildungsinformations-Nachweisabschnitt als Abbildungsinformation einen automatischen Belichtungswert, zum Beispiel einen Lichtmengenwert, oder eine Abbildungsvergrößerung zum Aufnehmen des Subjekts unter Verwendung des Abbildungsabschnitts (12, 26), oder Subjektinformation bezüglich einer Struktur und einer Komponente des lebenden Körpers des Subjekts, die von dem Abbildungsabschnitt aufgenommen wurden, nachweist,
wobei das von dem ersten Lichtquellenabschnitt (34) emittierte schmalbandige Licht eine ausgezeichnete Nachweisbarkeit für die Struktur und die Komponente in dem lebenden Körper des Subjekts aufweist, verglichen mit dem von dem zweiten Lichtquellenabschnitt (32, 24) emittierten breitbandigen Licht, und
wobei die Lichtemissionsbedingungen des ersten Lichtquellenabschnitts (34) und des zweiten Lichtquellenabschnitts (32, 24) und eine Bildverarbeitungsbedingung des Bildverarbeitungsabschnitts (36) derart geändert werden, dass Nachweis- und Hervorhebungsmaß der Struktur und der Komponente des lebenden Körpers des Subjekts basierend auf der von dem Abbildungsinformations-Nachweisabschnitt (56) nachgewiesenen Abbildungsinformation geändert werden.

2. Endoskopvorrichtung nach Anspruch 1, weiterhin umfassend:
einen Lichtemissionsverhältnis-Änderungsabschnitt, der Lichtemissionsverhältnisse des von dem ersten Lichtquellenabschnitt emittierten schmalbandigen Lichts und des von dem zweiten Lichtquellenabschnitt emittierten breitbandigen Lichts ändert, um die Lichtemissionsbedingugnen des ersten Lichtquellenabschnitts und des zweiten Lichtquellenabschnitts zu ändern.

3. Endoskopvorrichtung nach Anspruch 2, bei der die Abbildungsinformation in dem automatischen Belichtungswert besteht, und
wobei der Lichtemissionsverhältnis-Änderungsabschnitt ein Lichtemissionsverhältnis des von dem ersten Lichtquellenabschnitt emittierten schmalbandigen Lichts erhöht, wenn der automatische Belichtungswert klein ist, und ein Lichtemissionsverhältnis des von dem zweiten Lichtquellenabschnitt emittierten breitbandigen Lichts erhöht, wenn der automatische Belichtungswert groß ist.

4. Endoskopvorrichtung nach Anspruch 2, bei dem die Abbildungsinformation in der Abbildungsvergrößerung besteht, und
wobei der Lichtemissionsverhältnis-Änderungsabschnitt ein Lichtemissionsverhältnis des von dem ersten Lichtquellenabschnitt emittierten schmalbandigen Lichts erhöht, wenn die Abbildungsvergrößerung groß ist, und das Lichtemissionsverhältnis des von dem zweiten Lichtquellenabschnitt emittierten breitbandigen Lichts erhöht, wenn die Abbildungsvergrößerung klein ist.

5. Endoskopvorrichtung nach einem der Ansprüche 2 bis 4, bei dem, wenn die Lichtemissionsverhältnisse von dem Lichtemissionsverhältnis-Änderungsabschnitt geändert werden, mindestens eine der Größen elektrische Verstärkung des Abbildungsabschnitts, Abbildungszeit und Farbtoneinstellung der Abbildungsverarbeitung basierend auf den Lichtemissionsverhältnissen derart geändert wird, dass ein Weißabgleich des aufgenommenen Bilds nicht geändert wird.

6. Endoskopvorrichtung nach einem der Ansprüche 2 bis 5, bei der, wenn die Lichtemissionsverhältnisse von dem Lichtemissionsverhältnis-Änderungsabschnitt geändert werden, mindestens einer der Werte der elektrischen Verstärkung des Abbildungsabschnitts, eine Abbildungszeit und eine Farbtoneinstellung der Abbildungsverarbeitung basierend auf den Lichtemissionsverhältnissen derart geändert wird, dass eine Helligkeit des aufgenommenen Bilds nicht geändert wird.

7. Endoskopvorrichtung nach einem der Ansprüche 1 bis 6, bei der der Bildverarbeitungsabschnitt einen Bildhervorhebungsabschnitt enthält, der eine Frequenz-Hervorhebungskennlinie des aufgenommenen Bilds basierend auf der Abbildungsinformation ändert.

8. Endoskopvorrichtung nach Anspruch 7, bei der der Bildhervorhebungsabschnitt einen Frequenzband-Hervorhebungsabschnitt enthält, der zwei oder mehr Frequenzbänder des aufgenommenen Bilds hervorhebt, und
wobei der Frequenzband-Hervorhebungsabschnitt die Frequenz-Hervorhebungskennlinie einschließlich einer Änderung in einem hervorzuhebenden Frequenzband basierend auf der Abbildungsinformation ändert.

9. Endoskopvorrichtung nach Anspruch 8, bei der die Abbildungsinformation der automatische Belichtungswert ist, und wobei der Frequenzband-Hervorhebungsabschnitt das hervorzuhebende Frequenzband zur niederfrequenten Seite hin nach Maßgabe einer Zunahme des automatischen Belichtungswerts ändert.

10. Endoskopvorrichtung nach Anspruch 8 oder 9, bei der die Abbildungsinformation der automatische Belichtungswert ist,
wobei das von dem Frequenzband-Hervorhebungsabschnitt hervorgehobene Frequenzband eine Bandpasskennlinie ist, und
wobei der Frequenzband-Hervorhebungsabschnitt das hervorzuhebende Frequenzband so ändert, dass eine Breite des hervorzuhebenden Frequenzbands vergrößert wird, wenn der automatische Belichtungswert einen ersten vorbestimmten Schwellenwert übersteigt.

11. Endoskopvorrichtung nach einem der Ansprüche 8 bis 10, bei der die Abbildungsinformation der automatische Belichtungswert ist, und
wobei der Frequenzband-Hervorhebungsabschnitt dem hervorzuhebenden Frequenzband ermöglicht, eine Bandpasskennlinie anzunehmen, wenn der automatische Belichtungswert ein zweiter vorbestimmter Wert oder ein darunter liegender Wert ist, und das hervorzuhebende Frequenzband so ändert, dass es eine Hochpasskennlinie annimmt, wenn der automatische Belichtungswert den zweiten vorbestimmten Wert übersteigt.

12. Endoskopvorrichtung nach Anspruch 8, bei der die Abbildungsinformation die Abbildungsvergrößerung ist, wobei der Frequenzband-Hervorhebungsabschnitt das hervorzuhebende Frequenzband zur höherfrequenten Seite hin nach Maßgabe einer Zunahme der Abbildungsvergrößerung ändert.

13. Endoskopvorrichtung nach Anspruch 7, bei der die Abbildungsinformation die Subjektinformation bezüglich einer Größe eines bräunlichen Bereichs oder einer Dicke eines Blutgefäßes ist, und
wobei der Bildhervorhebungsabschnitt die Frequenz-Hervorhebungskennlinie des aufgenommenen Bilds basierend auf der Größe des bräunlichen Bereichs oder der Dicke des Blutgefäßes ändert.

14. Endoskopvorrichtung nach Anspruch 13, bei der der Bildhervorhebungsabschnitt einen Frequenzband-Hervorhebungsabschnitt enthält, der zwei oder mehr Frequenzbänder des aufgenommenen Bilds hervorhebt, und
wobei der Frequenzband-Hervorhebungsabschnitt die Frequenz-Hervorhebungskennlinie einschließlich einer Änderung in einem hervorzuhebenden Frequenzband basierend auf der Größe des bräunlichen Bereichs oder der Dicke des Blutgefäßes ändert.

15. Endoskopvorrichtung nach Anspruch 14, bei der der Frequenzband-Hervorhebungsabschnitt das hervorzuhebende Frequenzband zur Seite höherer Frequenz nach Maßgabe einer Abnahme der Dicke des Blutgefäßes ändert.

16. Endoskopvorrichtung nach Anspruch 14, bei der der Frequenzband-Hervorhebungsabschnitt dem hervorzuhebenden Frequenzband ermöglicht, eine Bandpasskennlinie anzunehmen, wenn die Größe des bräunlichen Bereichs einer vorbestimmten Größe oder einem darunter liegenden Wert entspricht, und das hervorzuhebende Frequenzband derart ändert, dass eine Breite des hervorzuhebenden Frequenzbands vergrößert wird, wenn die Größe des bräunlichen Bereichs die vorbestimmte Größe übersteigt.

17. Endoskopvorrichtung nach einem der Ansprüche 1 bis 16, bei der der Abbildungsinformations-Nachweisabschnitt die Abbildungsinformation aus dem aufgenommenen Bild nachweist.

18. Endoskopvorrichtung nach Anspruch 17, bei der der Abbildungsinformations-Nachweisabschnitt den automatischen Belichtungswert aus einer Helligkeit des aufgenommenen Bilds nachweist.

## Revendications

1. Dispositif d'endoscope (10) comprenant :
une première section de source de lumière (34) qui émet une lumière à bande étroite ayant une largeur de bande caractéristique rétrécie selon les caractéristiques spectrales des spectres d'émission d'une structure et une composante d'un corps vivant en tant que sujet, telle que l'épaisseur ou analogue d'un vaisseau sanguin ;
une seconde section de source de lumière (32, 24) qui émet une lumière blanche à large bande ayant une large bande de longueurs d'onde, plus large que ladite largeur de bande rétrécie et incluant une région visible ;
une section d'imagerie (12, 26) qui capture une image dudit sujet en utilisant la lumière renvoyée par ledit corps vivant après que ladite lumière à bande étroite et ladite lumière à large bande sont émises vers ledit sujet simultanément par ladite première section de source de lumière (34) et ladite seconde section de source de lumière (32), et fournit en sortie des informations d'image capturée ;
une section de traitement d'image (36) qui effectue un traitement d'image prédéterminé sur lesdites informations d'image capturée ; et
une section de détection d'informations d'imagerie (56) **caractérisée en ce que** ladite section de détection d'informations d'imagerie détecte en tant qu'informations d'imagerie une valeur d'exposition automatique, par exemple une valeur de quantité de lumière, ou un grossissement d'image pour capturer ledit sujet en utilisant ladite section d'imagerie (12, 26) ou des informations de sujet associées à une structure et une composante dudit corps vivant dudit sujet capturé par ladite section d'imagerie,
dans lequel ladite lumière à bande étroite émise par ladite première section de source de lumière (34) présente une excellente détectabilité pour la structure et la composante dudit corps vivant dudit sujet, par rapport à ladite lumière à large bande émise par ladite seconde section de source de lumière (32, 24), et
dans lequel les conditions d'émission de lumière de ladite première section de source de lumière (34) et de ladite seconde section de source de lumière (32, 24) et les conditions de traitement d'image de ladite section de traitement d'image (36) sont modifiées de façon à modifier les degrés de détection et d'accentuation de la structure et la composante dudit corps vivant dudit sujet sur la base desdites informations d'imagerie détectées par ladite section de détection d'informations d'imagerie (56).

2. Dispositif d'endoscope selon la revendication 1, comprenant en outre :
une section de modification de rapport d'émission de lumière qui modifie les rapports d'émission de lumière de ladite lumière à bande étroite émise par ladite première section de source de lumière et de ladite lumière à large bande émise par ladite seconde section de source de lumière, afin de modifier les conditions d'émission de lumière de ladite première section de source de lumière et de ladite seconde section de source de lumière.

3. Dispositif d'endoscope selon la revendication 2,
dans lequel lesdites informations d'imagerie sont constituées de ladite valeur d'exposition automatique, et
dans lequel ladite section de modification de rapport d'émission de lumière augmente le rapport d'émission de lumière de ladite lumière à bande étroite émise par ladite première section de source de lumière lorsque ladite valeur d'exposition automatique est petite et augmente le rapport d'émission de lumière de ladite lumière à large bande émise par ladite seconde section de source de lumière lorsque ladite valeur d'exposition automatique est grande.

4. Dispositif d'endoscope selon la revendication 2,
dans lequel lesdites informations d'imagerie sont constituées dudit grossissement d'image, et
dans lequel ladite section de modification de rapport d'émission de lumière augmente le rapport d'émission de lumière de ladite lumière à bande étroite émise par ladite première section de source de lumière lorsque ledit grossissement d'image est grand et augmente le rapport d'émission de lumière de ladite lumière à large bande émise par ladite seconde section de source de lumière lorsque ledit grossissement d'image est petit.

5. Dispositif d'endoscope selon l'une quelconque des revendications 2 à 4,
dans lequel, lorsque lesdits rapports d'émission de lumière sont modifiés par ladite section de modification de rapport d'émission de lumière, au moins une grandeur parmi le gain électrique de ladite section d'imagerie, la durée d'imagerie et le réglage de la teinte de couleur du traitement d'image est modifiée sur la base desdits rapports d'émission de lumière, de façon que l'équilibre des blancs de ladite image capturée ne soit pas modifié.

6. Dispositif d'endoscope selon l'une quelconque des revendications 2 à 5,
dans lequel, lorsque lesdits rapports d'émission de lumière sont modifiés par ladite section de modification de rapport d'émission de lumière, au moins une grandeur parmi le gain électrique de ladite section d'imagerie, la durée d'imagerie et le réglage de la teinte de couleur du traitement d'image est modifiée sur la base desdits rapports d'émission de lumière de façon que la luminosité de ladite image capturée ne soit pas modifiée.

7. Dispositif d'endoscope selon l'une quelconque des revendications 1 à 6,
dans lequel ladite section de traitement d'image comporte une section d'accentuation d'image qui modifie la caractéristique d'accentuation de fréquence de ladite image capturée sur la base des informations d'imagerie.

8. Dispositif d'endoscope selon la revendication 7,
dans lequel ladite section d'accentuation d'image comporte une section d'accentuation de bandes de fréquences qui accentue au moins deux bandes de fréquences de ladite image capturée, et
dans lequel ladite section d'accentuation de bandes de fréquences modifie ladite caractéristique d'accentuation de fréquence incluant une modification de la bande de fréquences à accentuer sur la base desdites informations d'imagerie.

9. Dispositif d'endoscope selon la revendication 8,
dans lequel lesdites informations d'imagerie sont constituées de ladite valeur d'exposition automatique, et
dans lequel ladite section d'accentuation de bandes de fréquences modifie ladite bande de fréquences à accentuer du côté des basses fréquences en fonction de l'augmentation de ladite valeur d'exposition automatique.

10. Dispositif d'endoscope selon la revendication 8 ou 9,
dans lequel lesdites informations d'imagerie sont constituées de ladite valeur d'exposition automatique,
dans lequel ladite bande de fréquences accentuée par ladite section d'accentuation de bandes de fréquences est une caractéristique passe bande, et
dans lequel ladite section d'accentuation de bandes de fréquences modifie ladite bande de fréquences à accentuer de façon à augmenter la largeur de ladite bande de fréquences à accentuer lorsque ladite valeur d'exposition automatique dépasse une première valeur prédéterminée.

11. Dispositif d'endoscope selon l'une quelconque des revendications 8 à 10,
dans lequel lesdites informations d'imagerie sont constituées de ladite valeur d'exposition automatique ;
dans lequel ladite section d'accentuation de bandes de fréquences permet à ladite bande de fréquences à accentuer d'avoir une caractéristique passe bande lorsque ladite valeur d'exposition automatique est inférieure ou égale à une seconde valeur prédéterminée, et modifie ladite bande de fréquences à accentuer de façon à avoir une caractéristique passe haut lorsque ladite valeur d'exposition automatique dépasse ladite seconde valeur prédéterminée.

12. Dispositif d'endoscope selon la revendication 8,
dans lequel lesdites informations d'imagerie sont constituées dudit grossissement d'image,
dans lequel ladite section d'accentuation de bandes de fréquences modifie ladite bande de fréquences à accentuer du côté des hautes fréquences en fonction de l'augmentation dudit grossissement d'image.

13. Dispositif d'endoscope selon la revendication 7,
dans lequel lesdites informations d'imagerie sont constituées desdites informations du sujet associées aux dimensions d'une région brunâtre ou à l'épaisseur d'un vaisseau sanguin, et
dans lequel ladite section d'accentuation d'image modifie ladite caractéristique d'accentuation de fréquence de ladite image capturée sur la base des dimensions de ladite région brunâtre ou de l'épaisseur dudit vaisseau sanguin.

14. Dispositif d'endoscope selon la revendication 13,
dans lequel ladite section d'accentuation d'image comporte une section d'accentuation de bandes de fréquences qui accentue au moins deux bandes de fréquences de ladite image capturée, et
dans lequel ladite section d'accentuation de bandes de fréquences modifie ladite caractéristique d'accentuation de fréquence incluant une modification de la bande de fréquences à accentuer sur la base des dimensions de la région brunâtre ou de l'épaisseur du vaisseau sanguin.

15. Dispositif d'endoscope selon la revendication 14,
dans lequel ladite section d'accentuation de bandes de fréquences modifie ladite bande de fréquences à accentuer du côté des hautes fréquences en fonction de la diminution de l'épaisseur dudit vaisseau sanguin.

16. Dispositif d'endoscope selon la revendication 14,
dans lequel ladite section d'accentuation de bandes de fréquences permet d'accentuer ladite bande de fréquences de manière à avoir une caractéristique passe bande lorsque les dimensions de ladite région brunâtre sont inférieures ou égales à des dimensions prédéterminées et modifie ladite bande de fréquences à accentuer de façon à augmenter la largeur de ladite bande de fréquences à accentuer lorsque les dimensions de ladite région brunâtre dépassent lesdites dimensions prédéterminées.

17. Dispositif d'endoscope selon l'une quelconque des revendications 1 à 16,
dans lequel ladite section de détection d'informations d'imagerie détecte lesdites informations d'imagerie d'après ladite image capturée.

18. Dispositif d'endoscope selon la revendication 17,
dans lequel ladite section de détection d'informations d'imagerie détecte ladite valeur d'exposition automatique d'après la luminosité de ladite image capturée.
